# EUROPEAN PATENT APPLICATION

(11) **EP 4 678 089 A1**
(43) Date of publication of application: **14.01.2026**
(21) Application number: 25188697.4
(22) Date of filing: 10.07.2025
(51) Int. Cl.: A61B 1/00, H04N 23/55, A61B 1/05, A61B 1/045, H04N 23/50, A61B 1/04, H04N 13/00

(54) **IMAGING DEVICE WITH PIXILATED SHUTTER**

(30) Priority: 11.07.2024 US 202418769942
(71) Applicant: Karl Storz Imaging, Inc., Goleta, CA 93117 (US)
(72) Inventor: DUCKETT, III. George E., Goleta, 93117 (US)
(74) Representative: Weickert, Jonas

(57) **Abstract**

An imaging device (12) for obtaining an image of an interior of a body of a patient includes a lens assembly (18), a shutter (20) formed of a plurality of pixels (20a) and a controller (16) configured to actuate the plurality of pixels (20a) of the shutter (20) to define a plurality of zones, the plurality of zones including an inner zone (IZ) and an outer zone (OZ), each of the plurality of zones configured to be opened and closed. The controller (16) further actuates the shutter (20) so as to open and close the inner zone (IZ) and outer zone (OZ) to control the amount of light from the lens assembly (18) onto an image sensor (24).

## Description

### TECHNICAL FIELD

The disclosure relates to an imaging device for obtaining an image of an interior body of a patient.

### BACKGROUND

Three-dimensional stereoscopic imaging systems traditionally include a pair of cameras that are offset from each other so as to form two channels, e.g. a left channel and a right channel. Images captured by the cameras are processed by a controller and displayed with one image overlaid on the other as a three-dimensional (3D) stereo image. In particular, left and right image data are collected by an image sensor and image data from the image sensor is processed to generate a corresponding left and right image frames. However, two channel devices cannot maintain a constant horizon when rotated about the optical axis because the orientation of the channels relative to each other is fixed and the stereo disparity can only be along the direction of the channel separation, which rotates with the endoscope.

Accordingly, a single channel imaging system may be implemented which utilizes pupil splitting. However, the use of a single channel may create issues relating to the different image views. For instance, it is desirable to have image data with more light to facilitate image processing to generate high quality images. In other instances, it is desirable to have image data with less light to generate an image frame having greater field a depth.

In other aspects, the imaging system may be configured to generate white light and fluorescent light image frames. In cases where a fluorescent image frame is generated there is concern that too much light may obscure the fluorescent image.

Accordingly, it is desirable to have an imaging system which may adjust the light to generate high resolution images, generate images with a greater depth of field, and be conducive to fluorescent light.

Imaging devices and systems with shutters and/or for stereo imaging are known from prior art documents US8115993, US5914810, US6606113, US10365554 and EP0841586B1.

### SUMMARY

One aspect of the disclosure provides an imaging device for obtaining an image of an interior of a body of a patient. The imaging device includes a lens assembly, a shutter, an image sensor, and a controller. The lens assembly is configured to focus light. The shutter is formed of a plurality of pixels. The image sensor is configured to capture an image from the lens assembly and the shutter is configured to control the light transmitted to the image sensor. The controller includes a data processing hardware and a memory hardware, the memory hardware storing instructions that when executed on the data processing hardware cause the data processing hardware to perform operations. The controller is configured to actuate the plurality of pixels of the shutter to define a plurality of zones. The plurality of zones includes an inner zone and an outer zone. The outer zone is disposed on an outer periphery of the inner zone. Each of the plurality of zones is configured to be opened and closed. The controller further actuates the shutter so as to open and close the inner zone and outer zone so as to control the amount of light to and from the lens assembly directed onto the image sensor, wherein the inner zone and the outer zone are opened to provide a maximum amount of light and the outer zone is closed and the inner zone is open to reduce the amount of light.

The imaging device may be an endoscope or an exoscope and may be suitable for stereo imaging. The shutter can be configured to provide an adjustable aperture. The lens assembly may be or include an objective lens assembly. The shutter may be disposed infront of or within the lens assembly. The shutter may be an LCD (Liquid Crystal Display) shutter with adjustable pixels that can be switched to be transparent or opaque. Opening and closing the inner and outer zones may include changing the transparency of the pixels of the shutter so that light either passes the pixel of the shutter or is blocked, for example by controlling the pixels of an LCD. The controller may be a camera control unit. The shutter may comprise more than two zones.

In one aspect of the imaging device, the shutter includes a first half and a second half, and the plurality of zones further includes a first zone and a second zone, the first zone is disposed on the first half of the shutter and the second zone is disposed on the second half of the shutter. In such an aspect, the first half may be symmetrical to the second half. The two halves may define two apertures, for example a left and right aperture, for stereo imaging. Stereo imaging may be accomplished by alternatingly controlling the first half and the second half to be transparent or opaque. This allows to create a left and a right image that can be superposed to create a stereo image.

In one aspect, the imaging device includes an input for selecting between one of a greater depth of field mode and a greater image quality mode, the controller processing a selection of the greater depth of field mode to open the inner zone and close the outer zone and processing a selection of the greater image quality mode to open both the inner zone and outer zone. Closing the outer zone reduces the diameter of the aperture and increases depth of field. Opening the outer and the inner zone increases the diameter of the aperture and allows more light to reach the image sensor, thus increasing image quality. The input can be any means suitable for selecting the mode, such as a manual input in the form of a button, switch, touch screen or the like. The input can also be automatic and, for example, be triggered by the controller. The controller may provide the input based on an automatically selected imaging mode, a light condition, a state of the image (such as brightness or contrast) or the like. Decreasing the aperture defined by the shutter allows to increase depth of field. At the same time the intensity of illuminating light used to illuminate a scene observed by the imaging device may be increased, for example, to compensate for the smaller aperture of the shutter. This could also be done automatically and be controlled by the controller. Control of the shutter, the illuminating light and/or the image can be configured to use a feedback loop.

In one aspect, the imaging device includes a light source configured to provide a white light and an excitation light configured to generate a fluorescent light, wherein the controller is further configured to combine a white light image frame using one of the greater depth of field mode and the greater image quality mode and a fluorescent light image frame using the other of the greater depth of field mode and the greater image quality mode. In such an aspect, the controller may be configured to automatically open the inner zone and the outer zone when the fluorescent light is emitted and automatically open the inner zone and close the outer zone when white light is emitted. Different combinations of the state of the inner and outer zone of the shutter and the light emitted are possible. Control of the zones may be automatic and depend on the light provided by the light source.

In one aspect, the shutter may be rectangular. In another aspect, the zones may be rectangular. The zones may comprise segments of rectangular shape that can be controlled individually by the controller. The segments may be arranged in a grid pattern. This may allow for a simpler design of the shutter.

In one aspect, the imaging device includes an orientation detection unit configured to determine an orientation of the imaging device, the controller configured to process the orientation to open and close a pair of zones in the plurality of zones in an alternating manner, wherein the pair of zones correspond to the orientation. In such an aspect, the memory hardware further stores a desired orientation, and the controller is configured to process the orientation to open and close the pair of zones in the plurality of zones in an alternating manner, wherein the pair of zones correspond to the desired orientation. The orientation detection unit may determine the orientation, for example the rotational position, of the device using any known means such as a rotation sensor, magnetic sensor, acceleration sensor, gyroscope or image based means that view the imaging device or analyze the image recorded by the imaging device. Opening and closing the pair of zones in the determined orientation allows a left aperture and a right aperture to be created with a stereo base that corresponds to the current orientation of the imaging device. This allows to maintain an orientation of the stereo base regardless of the rotational position of the device by controlling the zones accordingly. Thus, the stereo image can be made to maintain an upright position or a position that corresponds to the desired orientation stored in the memory hardware.

In one aspect, each zone in the plurality of zones is wedge-shaped and arranged to form a circle. This allows to efficiently control the zones of the shutter, as the aperture of imaging devices is usually round.

In one aspect, the shutter is rectangular and the plurality of zones includes pairs of zones, wherein each of the pair of zones includes an inner zone and an outer zone disposed on a periphery of the inner zone and the controller is configured to selectively open and close the outer zone and the inner zone to change a depth of field of the first image and the second image. The zones may also be of a rectangular shape.

In yet another aspect of the disclosure, an imaging system for obtaining an image of an interior of a body of a patient is also provided. The imaging system includes an imaging device having a light source, a lens assembly, a shutter, and an image sensor. The imaging system further includes an input and a controller. The light source is configured to emit a white light and an excitation light configured to generate a fluorescent light. The lens assembly is configured to focus light. The shutter is formed of a plurality of pixels which are selectively opened and closed to define an inner zone and an outer zone, wherein the outer zone is disposed on a periphery of the inner zone and each of the inner zone and the outer zone includes a left portion and a right portion. The image sensor is configured to capture an image from the lens assembly. The shutter may be incorporated into the lens assembly. The input is configured to select between one of a greater depth of field mode and a greater image quality mode, wherein in the greater depth of field mode, the inner zone in one of the left portion and right portion inner zone is opened and the outer zone is closed in an alternating manner, and in the greater image quality mode, both the inner zone and the outer zone in the left portion and the right portion are opened in an alternating manner. The controller includes a data processing hardware and a memory hardware, the memory hardware storing instructions that when executed on the data processing hardware cause the data processing hardware to perform operations. These operations include processing the input to actuate the shutter in the greater depth of field mode and the greater image quality mode upon selection of the greater depth of field mode and the greater image quality mode, and combining an image frame generated in the greater depth of field mode with an image frame generated in the greater image quality mode during one of a white light operation and a fluorescent light operation. Aspects of the imaging device described above may also be aspects of the imaging system described herein. Combining image frames with a greater depth of field and a frames with a greater image quality allows to create images that have good image quality, such as good brightness and resolution, as well as an improved depth of field, to show more of the observed scene in focus.

In one aspect, the controller is configured to automatically open the inner zone and the outer zone when the fluorescent light is imaged or the excitation light is emitted and automatically open the inner zone and close the outer zone when white light is either emitted or imaged.

In one aspect, the controller is configured to combine a first portion of a surgical scene generated during the greater image quality mode under the fluorescent light operation with a second portion of the surgical scene generated during the greater depth of field mode under the white light operation, the first portion being a different image than the second portion.

In one aspect, the controller is configured to combine the image frame generated in a greater depth of field mode with the image frame generated in the greater image quality mode wherein the portions of a surgical scene that is not fluoresced and generated in the greater depth of field mode is combined with portions of the surgical scene that is fluoresced and generated in the greater image quality mode. White light images are often brighter than fluorescence images. Opening the outer zone and the inner zone helps to achieve a maximum brightness for the fluorescence images, i.e. images that are recorded upon illumination with excitation light, and closing the outer zone reduces the brightness of the white light while also providing greater depth of field. Combining images of portions of a surgical scene with white light and with fluorescence light allows for a combined image that shows both white light and fluorescence while also optimizing image quality and depth of field as described above.

It should be noted that it may also be reasonable to reverse control of the outer and inner zones with regards to white light and excitation light emission, if in a surgical situation fluorescence light is preferably imaged with a greater depth of field and white light is supposed to be imaged with a greater image quality.

In one aspect, the imaging system further includes an orientation detection unit configured to determine an orientation of the imaging device, wherein the controller is configured to process the orientation to open and close a pair of zones in an alternating manner, wherein the pair of zones correspond to the orientation. In such an aspect, the memory hardware further stores a desired orientation, and the controller is configured to process the orientation to open and close the pair of zones in the plurality of zones in an alternating manner, wherein the pair of zones correspond to the desired orientation.

In one aspect, the inner zone and the outer zone include a plurality of zones, each of which are wedge-shaped, and the inner zone and the outer zone are arranged to form a circle.

In one aspect, the shutter is rectangular.

The details of one or more implementations of the disclosure are set forth in the accompanying drawings and the description below. Other aspects, features, and advantages will be apparent from the description and drawings, and from the claims.

### BRIEF DESCRIPTION OF THE DRAWINGS

The embodiments set forth in the drawings are illustrative and exemplary in nature and not intended to limit the subject matter defined by the claims. The following description of the illustrative embodiments can be understood when read in conjunction with the following drawings, where like structure is indicated with like reference numerals and in which:
FIG. 1 is a schematic view of a video imaging system according to one or more aspects described herein.
FIG. 2 is a schematic description of the video system including a camera control unit.
FIG. 3A is an illustrative view of a shutter generating a right eye image.
FIG. 3B is an illustrative view showing the optical data being blocked by the shutter shown in FIG. 3A.
FIG. 4A is an illustrative view of the shutter shown in FIG. 3A generating a left eye image.
FIG. 4B is an illustrative view showing the optical data being blocked by the shutter shown in FIG. 4A.
FIG. 5A is an illustrative view of the shutter shown in FIG. 3A with both the left and right halves open.
FIG. 5B is an illustrative view showing the shutter shown in FIG. 5A allowing all the light to pass.
FIG. 6A depicts an aspect of a shutter according to one or more embodiments herein, wherein the shutter includes 16 zones.
FIG. 6B depicts the zones of the shutters being opened and closed to correspond with a rotation of the imaging device.
FIG. 7A depicts an aspect of a shutter according to one or more embodiments herein, wherein the shutter includes an inner zone and an outer zone.
FIG. 7B depicts the inner zones and outer zones of the shutters being opened and closed to correspond with a rotation of the imaging device.
FIG. 7C depicts an aspect of the shutter configured in a greater depth of field mode.
FIG. 8A depicts an aspect of a shutter according to one or more embodiments herein, wherein the shutter is rectangular.
FIG. 8B depicts the zones of the shutters being opened and closed to correspond with a rotation of the imaging device.

### DETAILED DESCRIPTION OF PREFERRED EMBODIMENTS

Implementations herein are directed toward an imaging device including a lens assembly, a shutter formed of a plurality of pixels, an image sensor, and a controller, wherein the controller is configured to actuate the plurality of pixels of the shutter to define a plurality of zones, the plurality of zones including an inner zone and an outer zone, the outer zone disposed on an outer periphery of the inner zone, each of the plurality of zones configured to be opened and closed, wherein the controller actuates the shutter so as to open and close the inner zone and outer zone and control the amount of light to or from the lens assembly and onto the image sensor, wherein the inner zone and the outer zone are opened to provide a maximum amount of light and the outer zone is closed and the inner zone is opened to reduce the amount of light accordingly. The system may be configured to generate images with high image quality, images with a greater depth of field relative to the high quality images, and images conducive for fluorescence lighting.

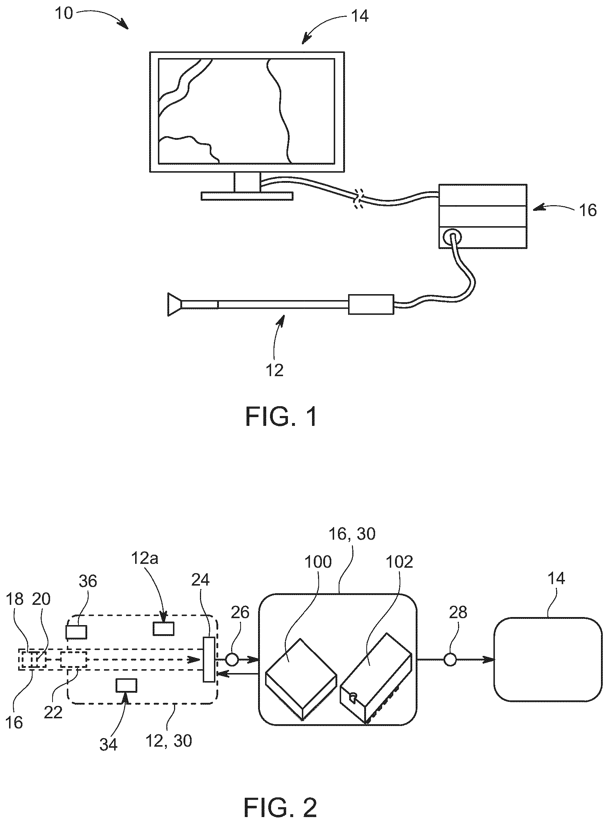

With reference now to FIG. 1, a schematic depiction of a video imaging system 10 is provided. For illustrative purposes, the video imaging system 10 is discussed in the context of a surgical procedure, but it should be appreciated that the system 10 may be used for other applications. The system 10 includes an imaging device 12 and a display 14. For illustrative purposes, the imaging device 12 is shown as a video endoscope and is coupled to a camera control unit 16. In one aspect, the imaging device 12 is detachably coupled to the camera control unit 16. The imaging device 12 is configured to capture images of an interior of a body of a patient and the camera control unit 16 is configured to perform image processing to generate a still or video image for production on the display 14.

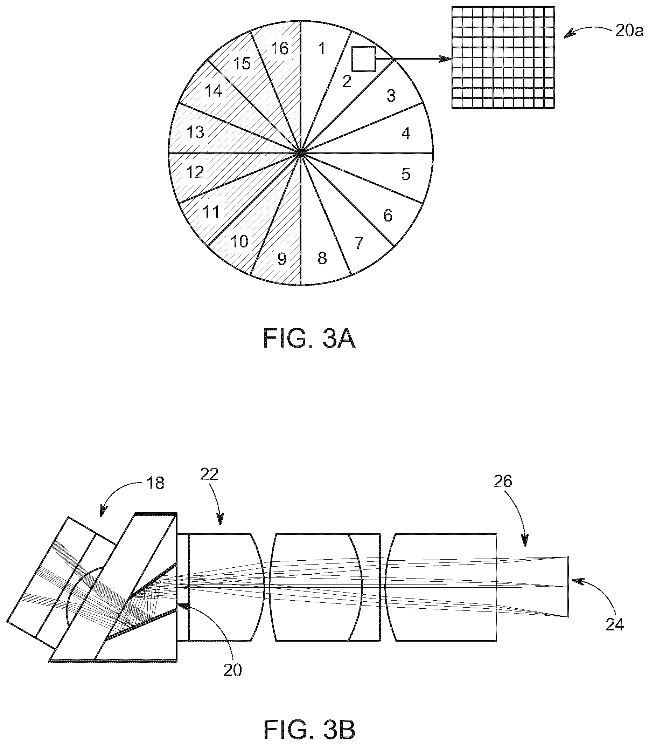

With reference now to FIGs. 2 and 3B, a depiction of the imaging device 12 is provided. The imaging device 12 includes a lens assembly 18 for collecting and focusing image light from a scene under observation and a shutter 20 configured to control the amount of light. For illustrative purposes, the shutter 20 is shown integrated within the lens assembly 18, but it should be appreciated that the shutter 20 may be formed on an outer surface of the lens assembly 18 or integrated within the lens assembly 18. In instances where the shutter 20 is formed on the outer surface of the lens assembly 18, a cover glass (not shown) may be placed on the shutter 20 so as to protect the shutter 20 from the environment. The lens assembly 18 may include a negative lens 18a disposed in front of the shutter 20 to help generate a wide field of view and a focusing lens group 22 configured to focus the image onto the image sensor 24. The imaging device 12 may further include a relay lens group configured to relay image light collected by the lens assembly 18 to an image sensor 24; however, it should be appreciated that relay lens group may be optional. In one aspect, the image sensor 24 may be a complementary metal oxide semiconductor (CMOS) or a Charged Coupled Device (CCD). It should be appreciated that any pixelated image sensor 24 currently known or later developed may be modified and adopted for use herein. The imaging device 12 may include a camera head unit 12a which is configured to control various camera functions such as the operation of the shutter 20, or move the lens assembly 18 to zoom in or out of the surgical scene.

In operation, the lens data 26 collected by the image sensor 24 is transmitted to the camera control unit (CCU) 16. The CCU 16 is coupled to the display 14, such as a monitor. The CCU 16 includes a data processing hardware 100 which executes instructions or programs stored on a memory hardware 102 for processing the lens data 26 from the image sensor 24 to generate an image frame 28 which is transmitted to the display 14 for view. It should be noted that the CCU 16 may be an element of the imaging device 12 or a separate unit as shown in FIG. 2. For example, the imaging device 12 may include the lens assembly 18, the shutter 20, the image sensor 24, the data processing hardware 100, and the memory hardware 102. Alternatively, the camera head 12a may be detachably connected to the imaging device 12 or integrally formed with the imaging device 12.

With reference again to FIG. 1, the video imaging device 12 is configured to capture image data from an image sensor 24 to generate a plurality of image frames 28 which may be compiled together to form a video image 30 for production on the display 14. As shown in FIGS. 3B, 4B and 5B, lens data 26 in the form of light is transmitted from a lens assembly 18 to the image sensor 24. The image sensor 24 processes the light into an image frame 28 in the form of an electric signal which is processed by the data processing hardware 100 disposed in the CCU 16. The CCU 16 includes the memory hardware 102 which stores instructions executable by the data processing hardware 100 which not only generates an image frame 28 but also performs image processing such as frame optimization, improved dynamic range, noise reduction, bandwidth filtering, edge detection, and the like. The image frames 28 are transmitted to the display 14 in the form of the video image 30.

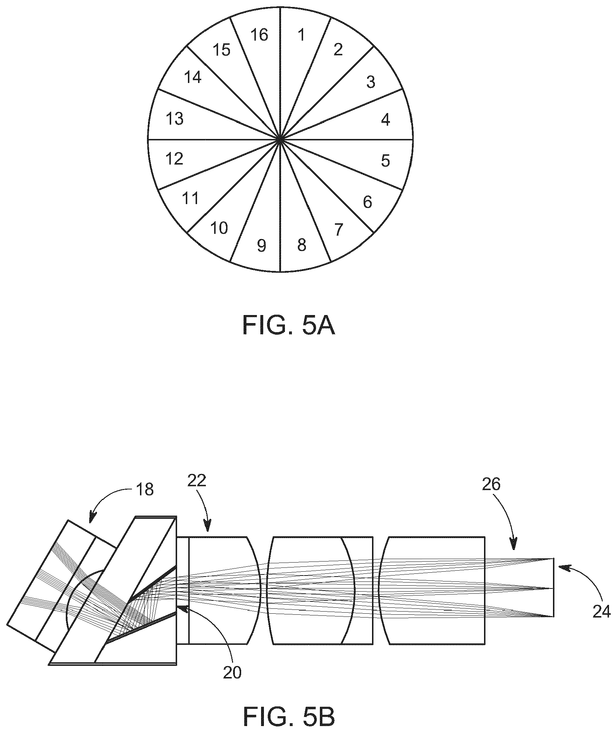

With reference now to FIGS. 3A-5B, a description of the shutter 20 is provided. In one aspect of the imaging device 12, the shutter 20 includes a first half and a second half. The shutter 20 is formed of a plurality of pixels 20a which may be turned on or off so as to define an aperture for light to flow. Each of the pixels 20a are connected to a power source (not shown) such as a battery housed within the camera control unit 16 or via a wired connection to a standard power outlet. When a pixel 20a is subjected to an electric current, the subjected pixel becomes opaque wherein light is blocked, and when a pixel 20a is not subjected to an electric current, the pixel is transparent. Any such shutter 20 currently known or used in the art or later developed may be modified for use herein, illustratively including a liquid crystal display shutter 20.

Accordingly, the amount of light that is passed through the shutter 20 and directed to the image sensor 24 may be controlled by turning on and off the pixels. A controller 30 is configured to actuate the shutter 20. As used herein, the term "controller" is used to describe the camera head 12a and/or the CCU 16 individually or collectively. That is, the camera functions executed by the controller 30 may be performed by the camera head 12a or the CCU 16, individually or collectively. Such functions not only include camera functions such as actuating the shutter 20 so as to selectively turn on or off the pixels, zooming, panning, changing the field of view, or operating a light source 36, but also image processing functions such as edge detection, contrast, color enhancement, and the like.

FIG. 3A illustrates an aspect of the shutter 20 wherein the shutter 20 is a generally planar member in the shape of a circle. The controller 30 is configured to actuate the pixels 20a to define a plurality of zones. Each zone forms a wedge, and for exemplary purposes, the shutter 20 is actuated to define sixteen (16) zones, each of which are number to facilitate a description of the operation. It should be appreciated that each zone may include the same number of pixels wherein each zone is similar in dimension to each other, may have a different number of pixels wherein each zone is different in dimension from each other or the zone may be a single pixel having a wedge shape. FIG. 3A depicts an aspect where eight of the zones define a right half and the other eight zones define a left half of the shutter 20. In such an aspect, the right half may be symmetrical to the left half.

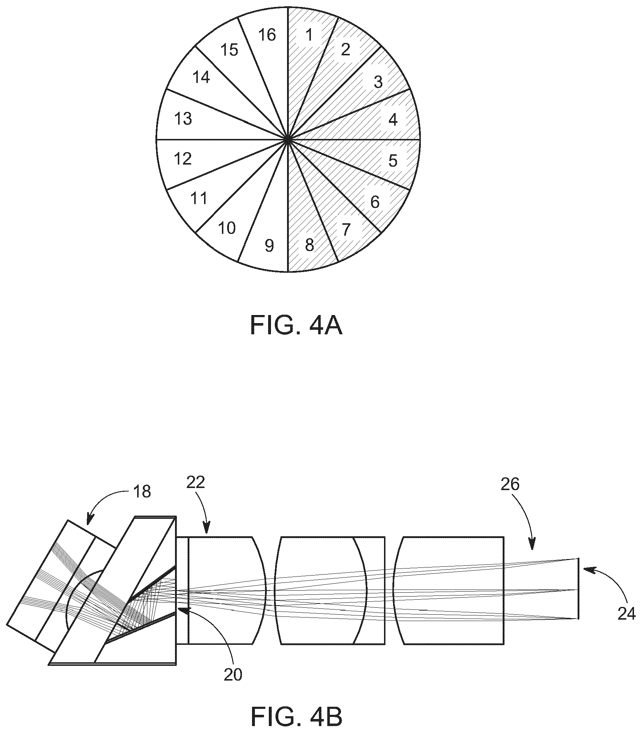

FIG. 3A illustrates a case where the controller 30 turns off eight (8) zones (numbered zones 1-8) so as to generate a right eye image. FIG. 3A illustrates the pixels 20a occupying the left half of the shutter 20 as shaded, indicating that they are turned on (e.g. subject to an electric current) so as to be opaque, blocking light from passing, and the pixels 20a occupying the right half of the shutter 20 are turned off so as to be transparent and allow light to pass. Accordingly, the lens data 26 processed by the controller 20 generates an image that mimics the view of a person's right eye, e.g. generates a right eye image. FIG. 4A illustrates a case where the controller 30 turns off eight (8) zones so as to generate a left eye image. FIG. 4B illustrates how the pixels 20a occupying the right half of the shutter 20 are turned on (e.g. subject to an electric current) so as to be opaque, blocking light from passing, and the pixels 20a occupying the left half of the shutter 20 are turned off so as to be transparent and allow light to pass. Accordingly, the lens data 26 processed by the CCU 16 generates an image that mimics the view of a person's left eye, e.g. a left eye image. The CCU 16 is further configured to process the right eye image and the left eye image to generate a three-dimensional image. Accordingly, the imaging device 12 is configured to generate a three-dimensional image using a single channel. FIG. 5A illustrates a case where the controller 30 turns off all of the zones. In such a mode, the CCU 16 processes the lens data 26 to generate a two-dimensional image of the surgical scene. In such an aspect, more light is provided to the surgical scene so as to facilitate the generation of a high quality two-dimensional image. As used herein, the term "high quality" means an image generated by processing more light as determined by the configuration of the shutter 20 and the image processing of the CCU 16. As is known to those skilled in the art, there is a trade-off between depth of field and image quality. Obtaining depth of field requires less light exposure; however, the aspects of the image such as edges and color may not appear as sharp or clear. On the other hand, increasing the light will make the edges and color appear more sharp or clear but the depth of field is degraded. In particular, "high quality" is used to describe an image frame 28 that is generated with more light relative to an image frame 28 where depth of field is desired. Accordingly, the imaging device 12 may be configured to provide a three-dimensional image and a two-dimensional image using a single channel. The imaging system 10 may further include an input 32 that may be configured to select between a three-dimensional image and a two-dimensional image.

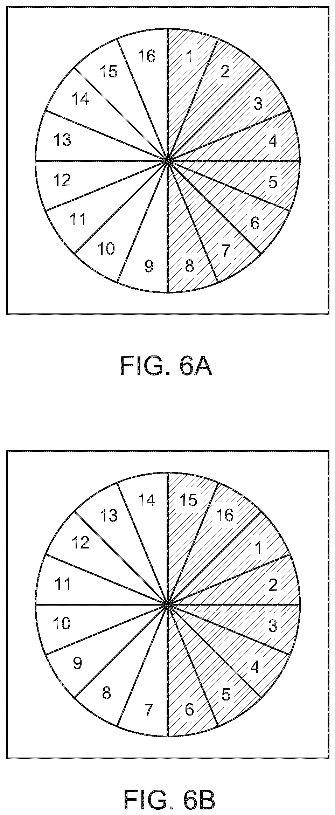

With reference now to FIGS. 6A and 6B, the imaging device 12 may be further configured to maintain a constant horizon. That is, the imaging device 12 may be configured to keep the image in a constant orientation through a rotation of the imaging device 12. In such an aspect, the imaging device 12 further includes an orientation detection unit 34 configured to determine an orientation of the imaging device 12. Any orientation detection unit 34 currently known or later developed may be modified for use herein, illustratively including a gyroscope. The controller 30 is configured to process the orientation detected by the orientation detection unit 34 to open and close a pair of zones that correspond to the orientation.

For illustrative purposes, the shutter 20 is shown as having sixteen zones, numbered 1-16. Eight of the zones are shaded, indicating that an electric current actuates each of the pixels occupying the eight shaded zones, and thus, the shaded zones are opaque, while the non-shaded zones are transparent as a result of the pixels in the non-shaded zones being turned off. FIG. 6A shows an aspect where zones 1-8 are turned on, in which case, a left eye image of an object (such as a surgical scene) is generated. To generate a right eye image, zones 9-16 are turned on and zones 1-8 are turned off, in which case a right eye image of the object is generated, wherein the CCU 16 processes the left eye image and the right eye image to generate a three-dimensional image.

In the event that the imaging device 12 is rotated, the zones are turned on and off to maintain the orientation. FIG. 6B shows an instance where the imaging device 12 has been rotated 45 degrees in a clockwise manner relative to the position of the imaging device 12 shown in FIG. 6A. The degree of rotation may be automatically detected by an orientation detection unit 34, and the CCU 16 processes the rotation to determine which of the zones to turn on and off to maintain a desired orientation. In the instant case, assume that the desired orientation is the orientation shown in FIG. 6A, wherein the CCU 16 determines that zones 15, 16 and 1-6 are to be turned on while turning off zones 7-14 so as to generate a left eye image in the same orientation as the orientation shown in FIG. 6A. To generate a right eye image, zones 7-14 are turned on, and zones 15, 16, and 1-6 are turned off, in which case, a right eye image of the object is generated, wherein the CCU 16 processes the left eye image and the right eye image to generate a three-dimensional image in the same orientation as described above with respect to FIG. 6A. In such an aspect, the input 32 may be configured to set a desired orientation, and the memory hardware 102 further stores the desired orientation wherein the controller 30 processes the detected orientation to open and close the zones in an alternating manner that correspond to the desired orientation.

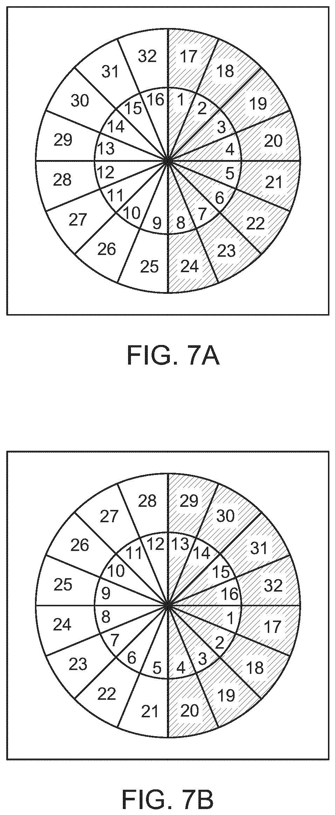

With reference now to FIGS. 7A-7C, in one aspect of the imaging device 12, the pixels of the shutter 20 may be actuated to define an inner zone "IZ" and an outer zone "OZ". The outer zone "OZ" is disposed on an outer periphery of the inner zone "IZ". The inner zone "IZ" and the outer zone "OZ" are configured to be opened and closed. For illustrative purposes, the shutter 20 is described as having thirty-two (32) zones that are number 1-32, wherein zones 1-16 form the inner zone "IZ" and zones 17-32 form the outer zone "OZ". FIG. 7A depicts the imaging device 12 generating a left eye image wherein the zones (1-8, 17-24) occupying the right half of the shutter 20 are turned on and the zones (9-16, 25-32) occupying the left half of the shutter 20 are turned off. To generate a right eye image, the imaging device 12 turns on the zones (9-16 and 25-32) occupying the left half of the shutter 20 and turns off the zones (1-8 and 17-24) occupying the right half of the shutter 20. To generate a three-dimensional image, the CCU 16 processes the left eye image and the right eye image.

FIG. 7B shows the imaging device 12 maintaining the orientation shown in FIG. 7A through a rotation of the imaging device 12. FIG. 7B shows an instance where the imaging device 12 has been rotated 90 degrees in a clockwise manner relative to the position of the imaging device 12 shown in FIG. 7A. Upon a 90 degree rotation, the CCU 16 turns on zones 1-4, 13-16, 29-32, and 17-20 while turning off zones 5-12 and 21-28 so as to generate a left eye image in the same orientation as the orientation shown in FIG. 7A. To generate a right eye image, zones 5-12 and 21-28 are turned on, and zones 1-4, 13-16, 29-32, and 17-20 are turned off, in which case, a right eye image of the object is generated, wherein the CCU 16 processes the left eye image and the right eye image to generate a three-dimensional image in the same orientation as described above with respect to FIG. 7A.

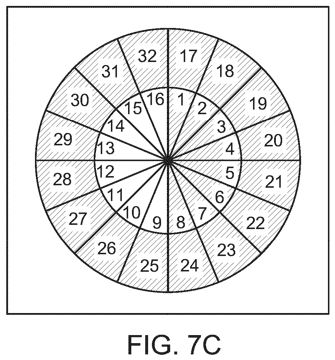

With reference now to FIG. 7C, the imaging device 12 may be further configured to adjust the amount of light so as to switch between a greater depth of field mode and a greater image quality mode. In such an aspect, the controller 30 actuates the shutter 20 to selectively open and close the inner zone "IZ" and outer zone "OZ" thereby controlling the amount of light from the lens assembly directed onto the image sensor 24. In particular, the inner zone "IZ" and the outer zone "OZ" are opened (e.g. the pixels are not turned on) to provide a maximum amount of light as shown in FIGs. 7A and 7B, and the outer zone "OZ" is closed and the inner zone "IZ" is selectively open to reduce the amount of light as shown in FIG. 7C.

The imaging device 12 may further include an input 32 for selecting between the greater depth of field mode and the greater image quality mode. The input 32 may be a button disposed on the camera head unit 12a, or may be a microphone, or a touch screen of a monitor, key board, or the like. It should be appreciated that the imaging device 12 may be configured such that the greater image quality mode is a default mode, in which case, the input 32 is configured to select or cancel the greater depth of field mode. The controller 30 processes a selection of the greater depth of field mode to open the inner zone "IZ" and close the outer zone "OZ" and processes a selection of the greater image quality mode to open both the inner zone "IZ" and outer zone "OZ". Thus, in the greater depth of field mode, the image sensor 24 receives less light relative to the greater image quality mode which facilitates image processing for generating a three-dimensional image with greater depth of field relative to the same image subjected to more light. Such a process is routine and known to those skilled in the art.

FIG. 7A depicts an aspect where the imaging device 12 is in a greater image quality mode for generating a three-dimensional image, wherein the shutter 20 is configured to generate a left eye view. In the greater image quality mode, a left eye image is obtained by turning off all of the zones (9-16 and 25-32) occupying the left half of the shutter 20, and turning on all of the zones occupying the right half of the shutter 20 (1-8 and 17-24). FIG. 7C depicts an aspect where the imaging device 12 is in the greater depth of field mode, wherein a left eye image is generated. As shown, the amount of light is reduced relative to the amount of light available in FIG. 7A. In particular, the outer zones (25-32) are turned on, thus light is only passed through the inner zones "IZ" occupying the left hand of the shutter 20. The imaging device 12 generates a right eye image by turning on all of the pixels in the outer zone "OZ" and the pixels in the inner zone "IZ" occupying the left half of the shutter 20, and turning off the pixels in the inner zone "IZ" occupying the right half of the shutter 20. These image frames 28 are processed by the CCU 16 to generate a three-dimensional image. Accordingly, it should be appreciated that the imaging device 20 captures a left eye image and a right eye image in an alternating manner and combines the left eye image and the right eye image to generate the three-dimensional image. Further, these images may be compiled by the CCU 16 to generate a three-dimensional video image.

It should be appreciated that the actuation of the zones may be tunable at a pixel level or at a zone level. As discussed above, the CCU 16 may be configured to perform image evaluation using known techniques such as edge detection, pixel intensity, image contrast, and the like. In instances where the CCU 16 determines that the image quality is below a predetermined standard, the individual zones (1-32) or individual pixels within a corresponding zone may be turned on or off to adjust the amount of light until the generated image reaches a predetermined standard. In another aspect, the user may control the amount of light in any given mode by actuating the input 32 so as to turn on and off individual pixels or zones.

In one aspect, the imaging device 12 includes a light source 36 configured to provide a white light and a light for exciting a chemical dye so that it emits fluorescent light. In such an aspect, the controller 30 is further configured to actuate the light source 36 to generate white light or excitation light configured to excite a fluorescent light, wherein the imaging device 12 captures white light images and fluorescent light images which are processed by the CCU 16 to generate white light image frames 28 and fluorescent image frames 28.

In one aspect, the CCU 16 is configured to combine an image frame 28 generated in the greater depth of field mode with an image frame 28 generated in the greater image quality mode. As discussed above, in the greater image quality mode, the inner zone "IZ" and the outer zone "OZ" are open, allowing for more light relative to the greater depth of field mode. In addition to providing greater image quality, too much white light may degrade the edges of the fluoresced portions of the surgical image. That is, though the image quality of the non-fluoresced portions is enhanced, the image quality of the fluoresced portion of the surgical scene may be diminished. Conversely, in the greater depth of field mode, less light is allowed to pass as a result of the outer zone "OZ" being turned on, i.e. being closed. While low white or background light is conducive to the image quality of the fluoresced portions of the surgical scene, low light results in less image quality of the non-fluoresced portions of the surgical image. The CCU 16 may be further configured to combine the image frame 28 generated in a greater depth of field mode with the image frame 28 generated in the greater image quality mode wherein the portions of the surgical scene that are fluoresced and generated in the greater image quality mode are combined with portions of the surgical scene that are not fluoresced and generated in the greater depth of field mode.

It should be appreciated that the CCU 16 may be further configured to combine portions of an image frame 28 generated in the greater depth of field mode and portions of an image frame 28 generated in the greater image quality mode during a fluorescence light operation wherein a first portion of the surgical scene has greater depth of field relative to a second portion of the surgical scene, and the second portion of the surgical scene has greater image quality, e.g. edges, contrast, sharpness, and the like, relative to the first portion of the surgical scene.

The controller 30 may be configured to automatically open the inner zone and the outer zone when the excitation light is emitted, and automatically open the inner zone and close the outer zone when white light is emitted. As discussed above, the controller 30 may be configured to selectively actuate the zones and/or the pixels to adjust the amount of light exposure of the surgical scene. Such an aspect may be performed in addition to combining the image frames 28 taken during the greater depth of field and greater image quality mode.

Various implementations of the systems and techniques described herein can be realized in digital electronic and/or lens circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" and "computer-readable medium" refer to any computer program product, non-transitory computer readable medium, apparatus and/or device (e.g., magnetic discs, objective lens disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

The processes and logic flows described in this specification can be performed by one or more programmable processors, also referred to as data processing hardware 100, executing one or more computer programs to perform functions by operating on input data and generating output. The processes and logic flows can also be performed by special purpose logic circuitry, e.g., an FPGA (field programmable gate array) or an ASIC (application specific integrated circuit). Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto objective lens disks, or objective lens disks. However, a computer need not have such devices. Computer readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto objective lens disks; and CD ROM and DVD-ROM disks. The processor and the memory can be supplemented by, or incorporated in, special purpose logic circuitry.

To provide for interaction with a user, one or more aspects of the disclosure can be implemented on a computer having a display device, e.g., a CRT (cathode ray tube), LCD (liquid crystal display) monitor, or touch screen for displaying information to the user and optionally a keyboard and a pointing device, e.g., a mouse or a trackball, by which the user can provide input to the computer. Other kinds of devices can be used to provide interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

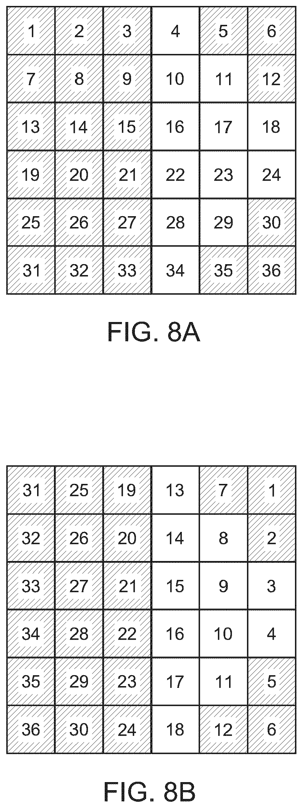

While particular embodiments have been illustrated and described herein, it should be understood that various other changes and modifications may be made without departing from the spirit and scope of the claimed subject matter. Moreover, although various aspects of the claimed subject matter have been described herein, such aspects need not be utilized in combination. It is therefore intended that the appended claims cover all such changes and modifications that are within the scope of the claimed subject matter. For example, FIGS. 8A and 8B show an aspect where the shutter 20 is rectangular. In such an aspect, the functions of the shutter 20 may be applied to any of the examples described herein. The shutter 20 in FIG. 8A includes 36 zones, each zone is shaped identical to the other and may include the same number of pixels. The zones that are shaded are turned on and thus are opaque and block light, whereas the clear zones are turned off and allow light to pass. FIG. 8B shows the imaging device 12 rotated 90 degrees relative to the imaging device 12 shown in FIG. 8A. FIG. 8B illustrates how the imaging device 12 may be configured to maintain the same orientation shown in FIG. 8A by actuating zones 13-18, 8-11, 3 and 4.

## Claims

1. An imaging device (12) for obtaining an image of an interior of a body of a patient, the imaging device (12) comprising:
a lens assembly (18) for focusing light;
a shutter (20) formed of a plurality of pixels (20a);
an image sensor (24) for capturing an image from the lens assembly (18), the shutter (20) controlling the light transmitted to the image sensor (24); and
a controller (16) including a data processing hardware (100) and a memory hardware (102), the memory hardware (102) storing instructions that when executed on the data processing hardware (100) cause the data processing hardware (100) to perform operations; and wherein
the controller (16) is configured to actuate the plurality of pixels (20a) of the shutter (20) to define a plurality of zones, the plurality of zones including an inner zone (IZ) and an outer zone (OZ), the outer zone (OZ) disposed on an outer periphery of the inner zone (IZ), each of the plurality of zones configured to be opened and closed, wherein the controller (16) further actuates the shutter (20) so as to open and close the inner zone (IZ) and outer zone (OZ) so as to control an amount of light from the lens assembly (18) onto the image sensor (24), wherein the inner zone (IZ) and the outer zone (OZ) is opened to provide a maximum amount of light and the outer zone (OZ) is closed and the inner zone (IZ) is open to reduce the amount of light.

2. The imaging device (12) as set forth in claim 1, wherein the shutter (20) includes a first half and a second half, and the plurality of zones further includes a first zone and a second zone, the first zone is disposed on the first half of the shutter (20) and the second zone is disposed on the second half of the shutter (20).

3. The imaging device (12) as set forth in claim 2, wherein the first half is symmetrical to the second half.

4. The imaging device (12) as set forth in any of claims 1 to 3, further including an input (32) for selecting between one of a greater depth of field mode and a greater image quality mode, the controller (16) processing a selection of the greater depth of field mode to open the inner zone (IZ) and close the outer zone (OZ) and processing a selection of the greater image quality mode to open both the inner zone (IZ) and outer zone (OZ).

5. The imaging device (12) as set forth in claim 4, further including a light source (36) configured to provide a white light and an excitation light configured to generate a fluorescent light, wherein the controller (16) is further configured to combine a white light image frame (28) using one of the greater depth of field mode and the greater image quality mode and a fluorescent light image frame (28) using the other of the greater depth of field mode and the greater image quality mode.

6. The imaging device (12) as set forth in claim 5, wherein the controller (16) is configured to automatically open the inner zone (IZ) and the outer zone (OZ) when the excitation light is emitted and automatically open the inner zone (IZ) and close the outer zone (OZ) when white light is emitted.

7. The imaging device (12) as set forth in any of the preceding claims, wherein the shutter (20) is rectangular.

8. The imaging device (12) as set forth in any of the preceding claims, further including an orientation detection unit (34) configured to determine an orientation of the imaging device (12), the controller (16) configured to process the orientation to open and close a pair of zones in an alternating manner, wherein the pair of zones correspond to the orientation.

9. The imaging device (12) as set forth in claim 8, wherein the memory hardware (102) further stores a desired orientation, the controller (16) configured to process the orientation to open and close the pair of zones in the plurality of zones in an alternating manner, wherein the pair of zones correspond to the desired orientation.

10. The imaging device (12) as set forth in claim 1, wherein each zone in the plurality of zones is wedge-shaped and arranged to form a circle.

11. The imaging device (12) as set forth in claim 8, wherein the shutter (20) is rectangular, and the plurality of zones is a pair of zones, each of the pair of zones includes an inner zone (IZ) and an outer zone (OZ) disposed on a periphery of the inner zone (IZ), the controller (16) configured to selectively open and close the outer zone (OZ) and the inner zone (IZ) to change a depth of field.

12. An imaging system (10) for obtaining an image of an interior of a body of a patient, the imaging system (10) comprising:
a light source (36) configured to transmit a white light and an excitation light;
an imaging device (12) including a lens assembly (18), a shutter (20) and an image sensor (24), the lens assembly (18) for focusing light, the shutter (20) being formed of a plurality of pixels (20a) which are selectively opened and closed to define an inner zone (IZ) and an outer zone (OZ), the outer zone (OZ) disposed on the periphery of the inner zone (IZ), the inner zone (IZ) and the outer zone (OZ) including a left portion and a right portion, and the image sensor (24) configured to capture an image from the lens assembly (18), wherein the shutter (20) is configured to control the amount of light transmitted to the image sensor (24);
an input (32) for selecting between one of a greater depth of field mode and a greater image quality mode, wherein in the greater depth of field mode the inner zone (IZ) in one of the left portion and right portion is opened and the outer zone (OZ) is closed, and wherein the inner zone (IZ) in the left portion and right portion are opened and closed in an alternating manner so as to generate a left and a right image, and in the greater image quality mode the both the inner zone (IZ) and the outer zone (OZ) are opened in an alternating manner so as to generate a left image and a right image; and
a controller (16) including a data processing hardware (100) and a memory hardware (102), the memory hardware (102) storing instructions that when executed on the data processing hardware (100) cause the data processing hardware (100) to perform operations to include processing the input (32) to actuate the shutter (20) in the greater depth of field mode and the greater image quality mode upon selection of the greater depth of field mode and the greater image quality mode and combine an image frame (28) generated in the greater depth of field mode with an image frame (28) generated in the greater image quality mode during one of a white light operation and fluorescent light operation.

13. The imaging system (10) as set forth in claim 12, wherein the controller (16) is configured to automatically open the inner zone (IZ) and the outer zone (OZ) when the excitation light is emitted and automatically open the inner zone (IZ) and close the outer zone (OZ) when white light is emitted.

14. The imaging system (10) as set forth in claim 12 or 13, wherein the controller (16) is configured to combine a first portion of a surgical scene generated during the greater image quality mode under the fluorescent light operation with a second portion of the surgical scene generated during the greater depth of field mode under the white light operation, the first portion being a different image than the second portion.

15. The imaging system (10) as set forth in any of claims 12 to 14, wherein the controller (16) is configured to combine the image frame (28) generated in a greater depth of field mode with the image frame (28) generated in the greater image quality mode wherein the portions of a surgical scene that is fluoresced and generated in the greater image quality mode is combined with portions of the surgical scene that is not fluoresced and generated in the greater depth of field mode.

16. The imaging system (10) as set forth in any of claims 12 to 15, further including an orientation detection unit (34) configured to determine an orientation of the imaging device (12), the controller (16) configured to process the orientation to open and close a pair of zones in an alternating manner, wherein the pair of zones correspond to the orientation.

17. The imaging system (10) as set forth in claim 16, wherein the memory hardware (102) further stores a desired orientation, the controller (16) configured to process the orientation to open and close a pair of zones in an alternating manner, wherein the pair of zones correspond to the desired orientation.

18. The imaging system (10) as set forth in any of claims 12 to 17, wherein the inner zone (IZ) and the outer zone (OZ) includes a plurality of zones each of which are wedge-shaped and the inner zone (IZ) and the outer zone (OZ) are arranged to form a circle.

19. The imaging system (10) as set forth in any of claims 12 to 17, wherein the shutter (20) is rectangular.
